# EUROPÄISCHE PATENTSCHRIFT

(11) **EP 1 218 531 B1**
(45) Veröffentlichungstag und Bekanntmachung des Hinweises auf die Patenterteilung: **24.11.2004**
(21) Anmeldenummer: 00965953.3
(22) Anmeldetag: 09.09.2000
(51) Int. Cl.: C12P 21/02, C12N 1/20

(54) **VERFAHREN ZUR GEWINNUNG VON BAKTERIORHODOPSIN**
METHOD FOR EXTRACTING BACTERIO-RHODOPSIN
PROCEDE D'EXTRACTION DE BACTERIORHODOPSINE

(30) Priorität: 24.09.1999 DE 19945798
(43) Veröffentlichungstag der Anmeldung: 03.07.2002
(73) Patentinhaber: MIB - Munich Innovative Biomaterials GmbH, 82152 Planegg-Martinsried (DE)
(72) Erfinder: KRISCHKE, Wolfgang, 72138 Kirchentellinsfurt (DE); BRUNNER, Herwig, 70569 Stuttgart (DE); SCHREINER, Lothar, 70563 Stuttgart (DE); OESTERHELT, Dieter, 82152 Martinsried (DE)
(74) Vertreter: Schrell, Andreas, Dr.
(86) Internationale Anmeldenummer: PCT/EP2000/008816
(87) Internationale Veröffentlichungsnummer: WO 2001/023415

(56) Entgegenhaltungen:
- EP-A- 0 286 869
- EP-A- 0 406 850
- FR-A- 2 590 273
- OESTERHELT D: "Isolation of purple membranes." 1995 , ARCHAEA: A LABORATORY MANUAL; HALOPHILES, PAGE(S) 55-57 , COLD SPRING HARBOR LABORATORY PRESS 10 SKYLINE DR., PLAINVIEW, NEW YORK 11803, USA XP000992317 ISBN: 0-87969-438-6 in der Anmeldung erwähnt Seite 55-57
- ABDULAEV N G ET AL.: "The solubilization and chemical characterization of bacteriorhodopsin" MEMBR. TRANSP. PROCESSES, Bd. 2, 1978, Seiten 53-57, XP000992416
- DATABASE WPI Section Ch, Week 200004 Derwent Publications Ltd., London, GB; Class B04, AN 2000-051067 XP002162995 & RU 2 115 722 C (AKSON STOCK CO), 20. Juli 1998 (1998-07-20)
- DATABASE WPI Section Ch, Week 199444 Derwent Publications Ltd., London, GB; Class D16, AN 1994-356669 XP002162997 & SU 1 824 440 A (BIOS COOP), 30. Juni 1993 (1993-06-30)
- DATABASE WPI Section Ch, Week 199415 Derwent Publications Ltd., London, GB; Class B04, AN 1994-125042 XP002162998 & SU 626 583 A (AS USSR BIOL PHYS INST), 15. Dezember 1993 (1993-12-15) in der Anmeldung erwähnt
- SUMPER M ET AL.: "Biosynthesis of the purple membrane of halobacteria" ANGEWANDTE CHEMIE INTERNATIONAL EDITION IN ENGLISH, Bd. 15, Nr. 4, 1976, Seiten 187-194, XP002162992 in der Anmeldung erwähnt

## Beschreibung

Die vorliegende Erfindung betrifft ein Verfahren zur Gewinnung von Bakteriorhodopsin aus Halobakterien.

Bakteriorhodopsin ist ein retinal-haltiges Membranpigment der Halobakterien. Bakteriorhodopsin wirkt als lichtabhängige, aus dem Zellinneren in die Umgebung gerichtete Protonenpumpe. Die Absorption eines Photons löst durch eine vorübergehende Konfigurationsänderung des Proteins den gerichteten Transport eines Protons zur Außenseite der Zelle aus. Hierdurch wechselt die Farbe des Proteins von violett nach gelb. Durch Aufnahme eines Protons aus dem Zellinneren wird innerhalb von wenigen Millisekunden der ursprüngliche Zustand wieder hergestellt. Das Bakteriorhodopsin-Molekül, das in zweidimensionaler kristalliner Anordnung in Form einer sogenannten Purpurmembran in der Zellmembran organisiert ist, stellt demgemäß also einen Licht-Energiewandler dar. Diese Eigenschaft und seine bemerkenswerte Stabilität im isoliertem Zustand eröffnen eine Vielzahl von technischen Nutzungen. So kann Bakteriorhodopsin in Form einer Purpurmembran als transienter optischer Speicher in der Datenverarbeitung, bei der Herstellung von Biosensoren oder Biochips und als elektrooptisches Schaltelement Verwendung finden. Der bei Belichtung entstehende elektrochemische Protonengradient könnte für eine makroskopische Ionenpumpe genutzt werden, z. B. auch zur Herstellung von molekularem Wasserstoff.

Eine wesentliche Voraussetzung für anwendungsnahe Forschungsarbeiten und kommerzielle Nutzung von Bakteriorhodopsin stellt jedoch die Verfügbarkeit größerer Bakteriorhodopsin-Mengen in möglichst großer Reinheit und mit möglichst geringem finanziellen Aufwand dar.

Die Synthese des Bakteriorhodopsins wird von vielen halophilen Archaea unter Kulturbedingungen wie Sauerstoffmangel und Licht induziert, wenn photosynthetisches Wachstum zur Alternative des aeroben Wachstums wird. Stämme sind verfügbar, die im Dunkeln und unter Sauerstofflimitation Bakteriorhodopsin konstitutiv exprimieren. Die am meisten verwendete Species Halobacterium salinarum benötigt für Wachstum Aminosäuren als C- und N-Quelle im Kulturmedium, so daß in der Regel Mediumsbestandteile wie Pepton verwendet werden müssen, allerdings mit einem stark negativen Einfluß auf die Mediumskosten. Da H. salinarum, im Batch-Verfahren im Bioreaktor gezüchtet, unter konstanter Belüftung nur am Ende der logarithmischen Phase Bakteriorhodopsin bildet, hatten die Oganismen bislang entsprechend niedrige Gesamtproduktivitäten. Aus Sumper et al. (Angewandte Chemie 88 (1976), 203 bis 210) und Oesterhelt (in: DasSarma S., Fleischmann E.M., Hrsg.: Archaea-Halophiles, a laboratory manual, Cold Spring Harbor Laboratory Press, New York 1995) sind Verfahren zur Bakteriorhodopsingewinnung mittels Zentrifugation zur Zellabtrennung, Dialyse und Dichtegradienten-Zentrifugation oder auch mittels Gelfiltration bekannt. Die beschriebenen Verfahren weisen jedoch eine Mehrzahl von Verfahrensschritten auf, die in unterschiedlichen Anlagen hintereinander durchgeführt werden müssen, so daß der Aufreinigungsprozess zeit- und materialaufwendig sowie arbeitsintensiv ist.

Eine weitere Schwierigkeit bei der Züchtung von Halobacterium salinarum ist die genetische Instabilität der bisher bekannten Laborstämme, d.h. die Fähigkeit zur Ausprägung von Bakteriorhodopsin kann im Verlauf der Vermehrung der Zellen verloren gehen und damit eine kontinuierliche Züchtung verbunden mit der Bildung von Bakteriorhodopsin unmöglich machen.

Die SU 626583 beschreibt die Kultivierung von halophilen Bakterien und deren Fermentation zur Gewinnung von Bakteriorhodopsin, wobei während der Kultivierung zusätzlich Licht eingestrahlt wird. Verfahren zur Extraktion von Bakteriorhodopsin aus halophilen Bakterien sind aus der SU 762392 bekannt. Die SU 1363778 beschreibt die Zugabe bestimmter Salze als Wachstumsstimulanzien für Halobacterium halobium. Aus der EP 0 406 850 sind Präparationsverfahren für Purpurmembranen, die Bakteriorhodopsin enthalten, bekannt. Schließlich beschreibt die CN 1 033 463 die Präparation und Immobilisierung von Bakteriorhodopsin aus Halobacterium halobium auf Festmembranen.

Keine der Offenbarungen ermöglicht jedoch eine kostengünstige und einfache Verfahrensweise, die mit geringem apparativem Aufwand Bakteriorhodopsin in möglichst großer Reinheit und Effizienz bereitstellt.

Der Erfindung liegt also das technische Problem zugrunde, ein Verfahren bereitzustellen, das in einfacher und kostengünstiger Weise die Gewinnung von Bakteriorhodopsin in hoher Ausbeute und Reinheit ermöglicht.

Die vorliegende Erfindung löst das ihr zugrundeliegende technische Problem durch die Bereitstellung eines Verfahrens zur Gewinnung von Bakteriorhodopsin aus Halobakterien, wobei die Halobakterien in einem Kulturmedium unter Zugabe von Hefeextrakt, hydrolysierten Eiweißfraktionen und/oder Aminosäure- oder Peptid-haltigen Stickstoffquellen einer Fermentation unterzogen werden, in einem anschließenden ersten Mikrofiltrationsschritt die Halobakterien und das Kulturmedium voneinander getrennt, die erhaltenen Halobakterien lysiert und in einem zweiten Mikrofiltrationsschritt niedermolekulare Zellbestandteile von einer bakteriorhodopsinhaltigen Fraktion abgetrennt werden. Die beiden Mikrofiltrationsschritte können vorzugsweise an ein und demselben Mikrofiltrationsmodul oder an zwei verschiedenen Mikrofiltrationsmodulen, ggf. mit unterschiedlicher Porenweite, durchgeführt werden.

Die erfindungsgemäße Verfahrenweise stellt eine besonders kostengünstige Möglichkeit zur Gewinnung von Bakteriorhodopsin dar, wobei in einfacher Art und Weise mit geringem apparativem Aufwand und der Möglichkeit einer unproblematischen Maßstabsvergrößerung die Gewinnung von Bakteriorhodopsin aus Halobakterien in großer Reinheit und Effizienz möglich ist. Die erfindungsgemäße Verfahrensweise eignet sich ausgezeichnet für einen kontinuierlichen Betrieb, wobei die Erfindung jedoch auch eine batchweise, diskontinuierliche oder semikontinuierliche beziehungsweise Repeated-Batch-Verfahrensweise ermöglicht. Überraschenderweise konnte gezeigt werden, daß die Verwendung von Hefeektrakt und/oder hydrolysierten Eiweißfraktionen als Stickstoff- bzw. Aminosäurequelle eine zumindest gegenüber dem üblicherweise verwendeten Pepton gleichbleibende, in den meisten Fällen aber verbesserte Bakteriorhodopsin-Produktivität ermöglicht, wobei jedoch vorteilhafterweise die Rohstoffkosten von Hefeextrakt nur ein Zehntel der Kosten für Pepton betragen. Erfindungsgemäß enthält das erfindungsgemäß eingesetzte Kulturmedium kein Pepton.

Im Zusammenhang mit der vorliegenden Erfindung werden unter Halobakterien Archaebakterien verstanden, die in stark salzhaltigen Flüssigkeiten, beispielsweise in Salzseen, vorkommen. Halobakterien zeichnen sich durch ihren Bedarf an hohen Kochsalzkonzentrationen in dem sie umgebenden Medium ebenso wie ihre hohen Salzkonzentrationen im Zellinneren sowie das Fehlen eines Murein-Sacculus aus. Typische Vertreter der Halobakterien sind Halobacterium salinarium und Halobacterium cutirubrum.

In besonders bevorzugter Ausführungsform erfaßt die Erfindung die Verwendung von Halobacterium salinarum. Die Erfindung erfaßt auch Mutanten, rekombinante Stämme oder sonstige, z.B. transgene, Wirtsorganismen, die sich zur Bildung von Bakteriorhodopsin eignen.

Im Zusammenhang mit der vorliegenden Erfindung wird unter Bakteriorhodopsin ein Chromoprotein verstanden, welches lichtsensitives Retinal als Chromophor enthält. In einer bevorzugten Ausführungsform der vorliegenden Erfindung stammt das Bakteriorhodopsin aus Halobakterien, insbesondere deren Purpurmembran. Selbstverständlich erfaßt die Erfindung unter dem Begriff Bakteriorhodopsin auch Abwandlungen, Modifikationen, Derivate oder Bruchstücke beziehungsweise Fragmente des Bakteriorhodopsins sowie andere Rhodopsine, sofern es sich bei den letztgenannten um Chromoproteine mit lichtsensitivem Retinal als Chromophor handelt. Die Erfindung erfaßt sowohl natürliches als auch durch gentechnische Verfahren in geeigneten Wirtzellen hergestelltes Bakteriorhodopsin. Die Erfindung erfaßt demgemäß also auch durch Aminosäure-Mutationen, Deletionen, Insertionen, Inversionen oder Derivatisierungen erzeugte Abwandlungen bekannter Bakteriorhodopsine sowie Fusionen von Bruchstücken oder kompletter Bakteriorhodopsinmoleküle mit weiteren Proteinkomponenten wie Signalpeptiden oder anderen Peptiden oder Proteinen. Die Erfindung erfaßt auch Derivatisierungen von Bakteriorhodopsin mit Kohlenhydraten, Fetten oder sonstigen Molekülen.

Im Zusammenhang mit der vorliegenden Erfindung wird unter einer Fermentation eine Kultivierung verstanden, das heißt die Gesamtheit aller Reaktionen in einer Mikroorganismen-Kultur, die den Substratverbrauch, die Bildung von Produkten und/oder die Bildung von Biomasse umfassen.

Im Zusammenhang mit der vorliegenden Erfindung werden unter Hefeextrakt die löslichen Inhaltsstoffe von Hefezellen verstanden, insbesondere technischer Hefeextrakt oder Extrakt aus Futterhefen, Reinzuchthefeextrakte, Molkehefen- oder Bierhefeextrakte.

Im Zusammenhang mit der vorliegenden Erfindung werden unter hydrolysierten Eiweißfraktionen Eiweißhydrolysate verstanden, das heißt Hydrolyseprodukte von Proteinen. Erfindungsgemäß handelt es sich dabei insbesondere um Produkte einer alkalischen, sauren oder enzymatischen Hydrolyse. Erfindungsgemäß werden unter hydrolysierten Eiweißfraktionen auch solche Eiweißfraktionen verstanden, die durch Partialhydrolyse hergestellt wurden. Insbesondere sieht die Erfindung die Verwendung von Milcheiweiß als hydrolysierte Eiweißfraktion vor.

Sowohl der Hefeextrakt als auch die hydrolysierte Eiweißfraktion wird erfindungsgemäß als Stickstoff-, Aminosäure- und/oder Kohlenstoffquelle im Kulturmedium verwendet. Überraschenderweise können dadurch zumindest gleichbleibende, vorteilhafterweise aber höhere Wachstumsraten der Halobakterien erreicht werden. Demgemäß können die Halobakterien auch unter Zugabe anderer, vorzugsweise kostengünstiger, Aminosäure- oder Peptid-haltiger Stickstoffquellen fermentiert werden, z.B. Maisquellwasser oder Baumwollsamenwasser.

Die weiteren Bestandteile des Kulturmediums sowie deren Konzentrationen entsprechen den üblicherweise verwendeten Bestandteilen eines Halobakterium-Kulturmediums. Ein derartiges Kulturmedium kann beispielsweise Natriumchlorid, Magnesiumsulfat, Tri-Natriumcitrat, Kaliumchlorid und Pepton oder Hefeextrakt sowie Wasser enthalten, insbesondere bei einem pH-Wert von 7,0 bis 8,5, bevorzugt 7,2 - 7,5.

Die Fermentation wird bevorzugt in einem Temperaturbereich von 35 - 50°, insbesondere bei 37° C, durchgeführt.

Gemäß der Erfindung wird in einem ersten Mikrofiltrationsschritt eine Trennung des wäßrigen Kulturmediums von den Halobakterien durchgeführt, indem die die Bakterien enthaltende Kulturflüssigkeit durch ein Membranfiltermodul geleitet wird, welches die Halobakterien im Retentat zurückhält. Die abgetrennten und aufkonzentrierten Mikroorganismen werden in vorteilhafter Weise anschließend lysiert, beispielsweise durch Zugabe von Wasser, welches durch osmotisches Eindringen die Zelle zerstört oder durch enzymatische Lyse und/oder Ultraschallbehandlung. Die Erfindung sieht in einer bevorzugten Ausgestaltung vor, nach Durchlaufen eines optionalen Waschschrittes mit Wasser oder Puffer, die bei der Zellyse freigesetzte DNA durch Zugabe von DNase (Deoxyribonuclease) zu hydrolysieren. Anschließend kann ein weiterer Waschschritt mittels z. B. Wasser oder Puffer vorgesehen sein.

Die Erfindung sieht weiter vor, daß in einem zweiten Mikrofiltrationsschritt die nach der Lyse erhaltene Reaktionssuspension einem weiteren Membranfiltrationsschritt unterzogen wird, so daß die niedermolekularen Zellbestandteile unter mehrmaliger Zugabe von Wasser von den das Bakteriorhodopsin enthaltenden Purpurmembranfragmenten abgetrennt bzw. ausgewaschen werden.

Im Retentat verbleibt im wesentlichen die Bakteriorhodopsinhaltige Membranfraktion, die, insbesondere nach Aufnahme in einem geeigneten Puffer, in einer bevorzugten Ausführungsform der Erfindung in einem dritten Mikrofiltrationsschritt, vorzugsweise an einem Mikrofiltrationsmodul anderer, insbesondere größerer, Porenweite vorteilhafterweise in einen große Purpurmembranfragmente und in einem kleine Purpurmembranfragmente enthaltenden Anteil aufgetrennt werden kann.

Die Erfindung sieht also vor, daß die Mikrofiltration vorzugsweise in sukzessiv folgenden unterschiedlichen Schritten durchgeführt wird. Erfindungsgemäß besonders bevorzugt ist es dabei, Membranfiltermodule mit Membranen beziehungsweise Filtern unterschiedlicher Porenweiten einzusetzen, wobei Porenweiten von 2,0 bis 0,05 µm bevorzugt werden. In besonders bevorzugter Ausführungsform werden für den ersten und zweiten Mikrofiltrationsschritt Porenweiten von 0,2 µm und für den dritten Mikrofiltrationsschritt Porenweiten von 0,8 µm bevorzugt.

In einer weiteren bevorzugten Ausführungsform der Erfindung sind die Membranfiltermodule, die im Zusammenhang mit der vorliegenden Erfindung auch als Mikrofiltrationsmodule bezeichnet werden, als röhrenförmige Filtermodule, also Röhrenmodule, Flachmembranmodule oder Wickelmodule ausgeführt. Die Membranfilter können organische oder anorganische Membranfilter sein, die vorzugsweise in Cross-Flow-Technik (Tangentialflußfiltration), insbesondere statisch oder rotierend/oszillierend eingesetzt werden.

Im Zusammenhang mit der vorliegenden Erfindung wird unter Cross-Flow-Technik verstanden, die Filtration so durchzuführen, daß die zu filtrierende Lösung oder die Suspension tangential über die Membran geführt wird, d. h. senkrecht zur Durchtrittsrichtung der Flüssigkeit durch die Membran.

Im Zusammenhang mit der vorliegenden Erfindung wird mit dem Begriff Membran der Filter an sich bezeichnet, also beispielsweise eine Röhrenmembran oder Flachmembran, hergestellt beispielsweise aus Keramik, Polymer etc. Im Zusammenhang mit der vorliegenden Erfindung wird unter einem Modul die Anordnung der Membran in einem Gehäuse mit Zu- und Ablauf, Dichtungen, Flanschen usw. verstanden, wobei das Gehäuse z.B. aus Edelstahl oder Kunststoff hergestellt sein kann.

Erfindungsgemäß können besonders bevorzugt keramische Mikrofiltrationsmembranen mit 2,0 bis 0,05 µm Porengröße einsetzen.

Erfindungsgemäß bevorzugt ist ein Filtratfluß von etwa 30 l/m²h, wobei selbstverständlich auch höhere Raten eingesetzt werden können. Nach der erfindungsgemäß vorgesehenen Lyse der Zellen steigt der Filtratfluß beim Auswaschen der Zellbestandteile in einer Ausführungsform der Erfindung auf bis zu 100 l/m²h an. Erfindungsgemäß wird es durch diese Vorgehensweise ermöglicht, die Purpurmembran im Konzentrat zurückzuhalten und von Mediumsbestandteilen und niedermolekularen Zellbestandteilen zu reinigen.

Die Erfindung betrifft in einer weiteren bevorzugten Ausführungsform ein vorgenanntes Verfahren, wobei nach der Zugabe von Hefeextrakt in das einzusetzende Kulturmedium möglicherweise auftretende Ausfällungen von Calcium- und Magnesiumphosphaten entweder vor der Fermentation durch Sedimentation, Zentrifugation oder Filtration entfernt werden oder nach der Fermentation bei der Abtrennung des Bakteriorhodopsins durch eine Absenkung des pH-Wertes aufgelöst und über das Permeat ausgetragen werden.

Die Erfindung betrifft in einer besonders bevorzugten Ausführungsform ein vorgenanntes Verfahren, wobei während der Fermentation eine gezielte Belüftung erfolgt und wobei die Fermentation bei einem Sauerstoffpartialdruck <= 20%, vorzugsweise unter 10% stattfindet, also nach oben limitiert aerobe Bedingungen vorliegen.

In bevorzugter Ausführungsform wird der Sauerstoffpartialdruck durch eine Regelung der Belüftungsrate oder der Rührerdrehzahl eingestellt und gehalten, sobald die Kultur eine maximale Zelldichte erreicht hat. Erfindungsgemäß ist vorgesehen, daß eine besondere Beleuchtung der Mikroorganismen, beispielsweise durch weißes Licht, im Produktionsreaktor nicht erfolgt.

Die Erfindung sieht eine batchweise, diskontinuierliche, semikontinuierliche, das heißt repeatedbatch-weise oder kontinuierliche Verfahrensweise vor. Besonders bevorzugt ist die kontinuierliche Verfahrensweise, wodurch eine erheblich erhöhte Bakteriorhodopsin-Produktivität erreicht wird, beispielsweise eine Erhöhung von 1,9 BR/lh (BR ≅ ODml/l = OD₅₆₀ₘₘ x Küvettenvolumen (in ml)/Probevolumen (1); ODml x 0,42 = mg BR, also Bakteriorhodopsin) bei einer batchweisen Betriebsweise auf ca. 3,2 BR/lh bei kontinuierlicher Betriebsweise. Hinzu kommt eine nochmalige Erhöhung der Effizienz der kontinuierlichen Betriebsweise durch den Wegfall der bei einer batchweisen Betriebsweise notwendigen Rüstzeit.

Das erfindungsgemäße Verfahren, insbesondere die Fermentation, kann in Bioreaktortypen unterschiedlicher Bauart erfolgen. Erfindungsgemäß bevorzugt ist es, scherungsarme Schlaufenreaktoren mit niedrigen Energieeintrag einzusetzen.

Da alle bisher bekannten Laborstämme von Halobacterium salinarum zur genetischen Instabilität neigen, die eine kontinierliche Zucht erschwert bzw. unmöglich macht, stellt die vorliegende Erfindung in bevorzugter Ausführungsform eines der vorgenannten Verfahren bereit, bei dem als Ausgangsmaterial für die erfindungsgemäß bevorzugte limitiert aerobe Kultivierung der Halobakterien eine anaerobe Vorkultur von Halobakterien verwendet wird. Die Erfindung betrifft also Verfahren zur Gewinnung von Bakteriorhodopsin aus Halobakterien, wobei in einem ersten Schritt unter anaeroben oder mikroaerophilen Bedingungen eine phototrophe Kultur von Halobakterien hergestellt wird, die als Ausgangsmaterial für die anschließend stattfindende Fermentation unter limitiert aeroben Bedingungen dient. Hierbei wird das phototrophe Wachstum unter mikroaerophilen oder aneroben Bedingungen zur Herstellung von Inocula im 5 Liter-Maßstab genutzt. Phototrophes Wachstum von Halobacterium salinarum findet nur statt, wenn die Zellen eine ausreichende Menge Bakteriorhodopsin produzieren. Zellen, die durch genetische Instabilität das Bakteriorhodopsin codierende bop Gen inaktiviert haben, können nicht wachsen.

Stabilitätsuntersuchungen, die von Einzelzellen ausgehen, zeigen, daß eine Vermehrung einer Kultur von 1 auf 10¹⁶ Zellen unter aeroben Bedingungen zum Verlust der Bakteriorhodopsin-Synthese führt, während unter anaeroben Bedingungen der Versuch bei 1:10⁴⁰ abgebrochen wurde, als noch keine Zelle ohne Bakteriorhodopsin-Produktion nachgewiesen werden konnte. Wird nun als Ausgangsmaterial für eine kontinuierliche aerobe Kultur eine anaerobe Vorkultur (1 Liter) verwendet, wird deren Stabilität um den Faktor 10²⁴ erhöht. Dies reicht aus, um auch in Hefeextraktmedien eine praktisch beliebige Zellmenge zu erzeugen.

Die Erfindung sieht in einer besonders bevorzugten Ausführungsform vor, daß das Kulturmedium oder Bestandteile davon nach Abtrennung der Halobakterien, also das Permeat des ersten Mikrofiltrationsschrittes, nach Durchlaufen einer Mediumskonditionierung wieder in dem erfindungsgemäßen Verfahren als Kulturmedium für die Fermentation eingesetzt werden kann, also recycelt wird. Das erfindungsgemäße Verfahren läßt sich also hinsichtlich des eingesetzten Kulturmediums oder von Bestandteilen davon kreislaufförmig durchführen, weist gleichsam einen Kreislauf, eine Regeneration oder eine Rezirkulation des Kulturmediums auf, wodurch eine erhebliche Kostenersparnis sowie Verfahrensvereinfachung durch Wiederverwednung der Salze gegeben ist. In dem erfindungsgemäß besonders bevorzugt durchgeführten Schritt der Mediumskonditionierung werden Bestandteile, die für eine weitere Verwendung des Kulturmediums ungünstig und nachteilig sind, entfernt. So enthält zum Beispiel das verbrauchte Kulturmedium eine erhöhte Konzentration an Ammoniumionen, die durch beispielsweise Wasserdampfstrippung oder Pervaporation mit Membrankontaktoren verringert werden kann. Andere unerwünschte Stoffe können z. B. mittels Zentrifugation, Filtration, Ausfällung, Sedimentation etc. abgetrennt werden. Das in die Mediumskonditionierung eingespeiste, verbrauchte Medium kann nach der Konditionierung direkt wieder in den Fermentationsschritt eingespeist werden. Es kann in besonders bevorzugter Ausführung der Erfindung aber auch vorgesehen sein, dem regenerierten Kulturmedium nach der Abtrennung unerwünschter Stoffe beispielsweise durch Sedimentation, Filtration oder Zentrifugation, bestimmte Hilfstoffe und/oder Hefeextrakte und/oder hydrolysierte Eiweißfraktionen zuzusetzen.

Weitere vorteilhafte Ausgestaltungen der Erfindung können den Unteransprüchen entnommen werden.

Die Erfindung wird anhand der Figuren und des dazu gehörigen Ausführungsbeispiels näher erläutert.

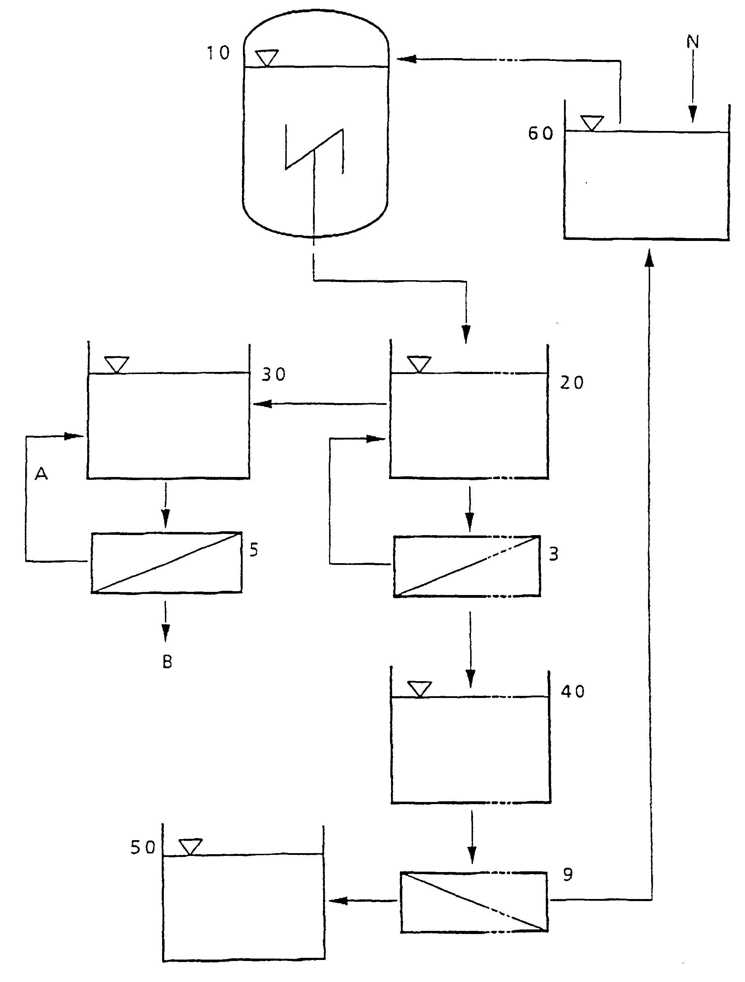

Die einzige Figur zeigt schematisch den Ablauf des erfindungsgemäßen Verfahrens.

Die Figur zeigt einen Bioreaktor 10, Puffertanks 20, 30, 40, 50 und Mikrofiltrationsmodule 3 und 5 sowie einen Zwischenbehälter für das Kulturmedium 60.

Der Bioreaktor 10 ist als 5 l MBR-Rührkesselreaktor mit einem Füllvolumen vor 4 l, pH- und Sauerstoffsonde, pH-Regulierung und pO₂-Regulierung von Hand über Luftdurchsatz ausgestattet. Bei kontinuierlichem Betrieb findet eine Füllstandsregulierung über das Reaktorgewicht und die Ablaufpumpe bei konstantem Mediumszufluß über eine Dosierpumpe (nicht dargestellt) statt.

Als Inoculum diente der Stamm "S9" Halobacterium salinarum, 200 mL (optische Dichte OD₅₇₈ₙₘ ca. 1,2.)

Das Medium war wie folgt zusammengesetzt: 250 g Natriumchlorid, 20 g Magnesiumsulfat, 3 g Tri-Natriumcitrat, 2 g Kaliumchlorid, 10 g Pepton L34 (Oxoid) oder Hefeextrakt Ohly KAT und Wasser auf 1 l Gesamtvolumen. Die pH-Regelung auf einen Wert von 7,5 erfolgte durch 1 M HCl bzw. 1 M NaOH. Zudem erfolgte Begasung mit Luft (40 l/h, 0, 17 vvm). Die Drehzahl des Blattrührers betrug 350 Upm. Es erfolgte eine Batch-Fermentation über ca. 90 Stunden bis zu einer OD₅₈₇ₙₘ von 1,5, wobei dann der Sauerstoff-Partialdruck < 15% betrug. Es wurde anschließend auf einen kontinuierlichen Betrieb mit einer Durchflußrate D = 0,02 l/h umgeschaltet, wobei der Sauerstoffpartialdruck unter 20% bleiben muß. Dies ergab in 300 h Gesamtlaufzeit 20 1 Fermentationsbrühe.

Nach der Fermentation werden die Halobakterien in einen Puffertank 20 verbracht, wo auch der Ablauf des Bioreaktors 10 im kontinuierlichen Betrieb aufgefangen wird. In einem ersten Filtrationsschritt an einem als Röhrenmodul ausgeführten Mikrofiltrationsmodul 3 werden die Halobakterien vom Kulturmedium abgetrennt, d.h. das Medium abgetrennt und die Bakterien um den Faktor 20 aufkonzentriert. Es wurde ein Keramik-Röhrenmodul Cerasiv mit 19 x 4 mm Kanälen, einer aktiven Fläche von 0,2 m² (Membranfläche) und 0,2 µm Porenweite eingesetzt. Es wurde eine Überstromgeschwindigkeit von 2 m/s, eine Filtrationsdauer von 4 Stunden bei ca. 30 l/m² h Abtrennleistung eingestellt. Im Puffertank 20 wird mit einem 5-fachen Volumen Wasser die Zelllyse durchgeführt und DNase (Fluka, Nr. 31135, 0,5 mg/l) hinzugegeben. Aus dem Puffertank 20 heraus werden in einem weiteren Mikrofiltrationsschritt an demselben Mikrofiltrationsmodul 3 die niedermolekularen Zellbestandteile durch Auswaschung im Permeat ausgetragen (Leitfähigkeit < 100 mS/cm²). Anschließend wird zweimal mit Wasser gewaschen bzw. filtriert. Im Retentat verbleiben die Purpurmembranfragmente, während die kleineren Zell- und Mediumsbestandteile abgetrennt werden. Die Abtrennleistung steigt im Lauf der Filtrationsschritte auf ca. 100 l/m²h. Demzufolge verringert sich der Zeitbedarf für die einzelnen Durchgänge immer weiter. Der Puffertank 30 dient zur Zwischenlagerung des Bakteriorhodopsin-haltigen Retentats. Durch Verwendung geeigneter Porenweite können die Bakteriorhodopsin-Membranfragmente weiter aufgetrennt werden, z.B. mittels eines Mikrofiltrationsmoduls 5 (keramisches Röhrenmodul, z.B. Cerasiv oder ATECH) mit einer Porenweite von z.B. 0,8 µm in einen hochmolekularen, relativ reinen Bakteriorhodopsinanteil A und in einen niedermolekularen Bakteriorhodopsinanteil B. Die Überströmgeschwindigkeit betrug 2 m/s.

Für den kontinuierlichen Betrieb des Bioreaktors sind zwei Tanks 20 erforderlich, einer zum Auffangen und einer zum Filtrieren bzw. Weiterverarbeiten.

Das im ersten Mikrofiltrationsschritt an dem Mikrofiltrationsmodul 3 abgetrennte salzhaltige Kulturmedium, also das Permeat, kann über einen Konditionierungsschritt weiter verwertet werden. Dazu werden in einem Behälter 40 Hilfsstoffe, Hefeextrakt und/oder hydrolysierte Eiweißfraktionen dem verbrauchten Medium hinzugegeben und eventuell schädliche Stoffe wie z. B. Ammoniumionen entfernt. Bei der Mediumskonditionierung auftretende Ausfällungen werden mittels einer Abtrennvorrichtung 9 beispielsweise mittels Sedimentation, Filtration oder Zentrifugation von dem regenerierten Kulturmedium abgetrennt. Die abgetrennten Ausfällungen werden in einem Behälter 50 gesammelt, während das regenerierte Kulturmedium R ggf. zusammen mit neuem Kulturmedium N in einem Zwischenbehälter 60 gelagert werden, um bei Bedarf in den Bioreaktor 10 eingeführt zu werden.

## Patentansprüche

1. Verfahren zur Gewinnung von Bakteriorhodopsin aus Halobakterien, wobei die Halobakterien in einem Hefeextrakt, hydrolysierte Eiweißfraktionen und/oder Aminosäure- oder Peptid-haltige Stickstoffquellen enthaltenden Kulturmedium einer Fermentation unterzogen werden und wobei in einem ersten Mikrofiltrationsschritt die Halobakterien und das Kulturmedium voneinander getrennt, die erhaltenen Halobakterien lysiert und in einem zweiten Mikrofiltrationsschritt niedermolekulare Zellbestandteile von dem Bakteriorhodopsin abgetrennt werden.

2. Verfahren nach Anspruch 1, wobei die Halobakterien durch Zugabe von Wasser lysiert werden.

3. Verfahren nach Anspruch 1 oder 2, wobei bei der Lyse der Halobakterien freigesetzte DNA durch Zugabe von DNase hydrolysiert wird.

4. Verfahren nach einem der Ansprüche 1 bis 3, wobei das gemäß Anspruch 1 erhaltene Bakteriorhodopsin in einem dritten Mikrofiltrationsschritt in große Purpurmembranfragmente und kleine Purpurmembranfragmente getrennt wird.

5. Verfahren nach einem der Ansprüche 1 bis 4, wobei nach der Zugabe von Hefeextrakt und vor der Fermentation eine Sedimentation, Zentrifugation oder Filtration durchgeführt wird.

6. Verfahren nach einem der Ansprüche 1 bis 5, wobei nach der Zugabe von Hefeextrakt und nach der Fermentation während der Abtrennung des Bakteriorhodopsins eine Absenkung des pH-Wertes durchgeführt wird.

7. Verfahren nach einem der Ansprüche 1 bis 6, wobei die Fermentation bei einem O₂-Partialdruck <= 20 % durchgeführt wird.

8. Verfahren nach einem der Ansprüche 1 bis 7, wobei im Anschluß an den ersten Mikrofiltrationsschritt zur Trennung des Bakteriorhodopsins vom Kulturmedium das abgetrennte Kulturmedium einem Konditionierungsschritt zugeführt wird.

9. Verfahren nach Anspruch 8, wobei das nach dem Konditionierungsschritt erhaltene regenerierte Kulturmedium den Halobakterien vor oder während der Fermentation zugesetzt wird.

10. Verfahren nach einem der Ansprüche 1 bis 9, wobei in den Mikrofiltrationsschritten Membranfiltermodule mit Membranen unterschiedlicher Porenweite eingesetzt werden.

11. Verfahren nach Anspruch 10, wobei die Membranfiltermodule röhrenförmige Filtermodule, Wickelmodule oder Flachmembranmodule sind.

12. Verfahren nach Anspruch 11, wobei die röhrenförmigen Filtermodule Hohlfasermodule oder Rohrmodule sind.

13. Verfahren nach einem der Ansprüche 1 bis 12, wobei Halobakterien der Art Halobacterium salinarum und davon abgeleitete Varianten, insbesondere Mutanten und rekombinante Stämme, verwendet werden.

14. Verfahren nach einem der Ansprüche 1 bis 13, wobei das Verfahren kontinuierlich, semikontinuierlich oder batchweise durchgeführt wird.

15. Verfahren nach einem der vorhergehenden Ansprüche, wobei der mindestens eine Mikrofiltrationsschritt in Cross-Flow-Technik ausgeführt wird.

16. Verfahren nach einem der vorhergehenden Ansprüche, wobei eine phototrophe Kultur von Halobakterien als Ausgangskultur für die Fermentation verwendet wird.

## Claims

1. Method for the recovery of bacteriorhodopsin from halobacteria, whereby the halobacteria are subjected to a fermentation within a culture medium containing yeast extract, hydrolysed protein fractions and/or nitrogen sources containing amino acid or peptide, and whereby the halobacteria and the culture medium are separated from one another during a first microfiltration stage, and whereby the resulting halobacteria are lysed, and whereby low molecular cell components are separated from the bacteriorhodopsin during a second microfiltration stage.

2. Method according to claim 1, whereby the halobacteria are lysed through an addition of water.

3. Method according to claim 1 or 2, whereby DNA released during the lysis of the halobacteria is hydrolysed through an addition of DNase.

4. Method according to one of the claims 1 to 3, whereby the bacteriorhodopsin extracted according to claim 1 is separated into large crimson membrane fragments and small crimson membrane fragments during a third microfiltration stage.

5. Method according to one of the claims 1 to 4, whereby a sedimentation, centrifugation, or filtration is carried out following the addition of yeast extract and prior to the fermentation.

6. Method according to one of the claims 1 to 5, whereby a lowering of the pH-value is carried out following the addition of yeast extract and following the fermentation during the separation of the bacteriorhodopsin.

7. Method according to one of the claims 1 to 6, whereby the fermentation is carried out at a partial O₂ pressure of <= 20%.

8. Method according to one of the claims 1 to 7, whereby the separated culture medium is extracted during a conditioning stage for the separation of the bacteriorhodopsin from the culture medium following the first microfiltration stage.

9. Method according to claim 8, whereby the regenerated culture medium obtained after the conditioning stage is added to the halobacteria prior to or during the fermentation.

10. Method according to one of the claims 1 to 9, whereby membrane filter modules with membranes of different pore widths are utilised during the microfiltration stages.

11. Method according to claim 10, whereby the membrane filter modules are tubular filter modules, wound modules, or flat membrane modules.

12. Method according to claim 11, whereby the tubular filter modules are hollow fibre modules or pipe modules.

13. Method according to one of the claims 1 to 12, whereby halobacteria of the type halobacteria salinarium and varieties derived from the same, especially mutants and recombinant strains, are utilised.

14. Method according to one of the claims 1 to 13, whereby the method is carried out continuously, semi-continuously, or in batches.

15. Method according to one of the preceding claims, whereby the at least one microfiltration stage is carried out with the aid of the cross flow technology.

16. Method according to one of the preceding claims, whereby a phototropic culture of halobacteria is utilised as the starting culture for the fermentation.

## Revendications

1. Procédé d'obtention de bactériorhodopsine à partir de bactéries halophiles, dans lequel les bactéries halophiles sont soumises à une fermentation dans un milieu de culture contenant un extrait de levure, des fractions d'albumine hydrolysé et/ou des sources d'azote contenant un acide aminé ou un peptide, et dans lequel, lors d'une première étape de microfiltration, on sépare l'un de l'autre les bactéries halophiles et le milieu de culture, on lyse les bactéries halophiles obtenues et, lors d'une seconde étape de microfiltration, on sépare les composants cellulaires de faible poids moléculaire de la bactériorhodopsine.

2. Procédé selon la revendication 1, dans lequel les bactéries halophiles sont lysées par ajout d'eau.

3. Procédé selon la revendication 1 ou 2, dans lequel, lors de la lyse des bactéries halophiles, l'ADN libéré est hydrolysé par ajout d'ADNase.

4. Procédé selon l'une des revendications 1 à 3, dans lequel la bactériorhodopsine obtenue selon la revendication 1 est séparée, lors d'une troisième étape de microfiltration, en grands fragments de membrane pourpre et en petits fragments de membrane pourpre.

5. Procédé selon l'une des revendications 1 à 4, dans lequel, après l'ajout d'extrait de levure et avant la fermentation, on réalise une sédimentation, une centrifugation ou une filtration.

6. Procédé selon l'une des revendications 1 à 5, dans lequel, après l'ajout d'extrait de levure et après la fermentation, pendant la séparation de la bactériorhodopsine, on réalise un abaissement de la valeur du pH.

7. Procédé selon l'une des revendications 1 à 6, dans lequel la fermentation est conduite avec une pression partielle de O₂ ≤ 20 %.

8. Procédé selon l'une des revendications 1 à 7, dans lequel, suite à la première étape de microfiltration pour séparer la bactériorhodopsine du milieu de culture, on amène le milieu de culture séparé vers une étape de conditionnement.

9. Procédé selon la revendication 8, dans lequel le milieu de culture régénéré obtenu après l'étape de conditionnement est ajouté aux bactéries halophiles avant ou pendant la fermentation.

10. Procédé selon l'une des revendications 1 à 9, dans lequel, lors des étapes de microfiltration, on utilise des modules à membranes filtrantes qui comportent des membranes de différentes porosités.

11. Procédé selon la revendication 10, dans lequel les modules à membranes filtrantes sont des modules filtrants tubulaires, des modules en roulés ou des modules à membranes planes.

12. Procédé selon la revendication 11, dans lequel les modules filtrants tubulaires sont des modules à fibres creuses ou à tubes.

13. Procédé selon l'une des revendications 1 à 12, dans lequel on utilise des bactéries halophiles du genre Halobacterium salinarium et ses variantes, en particulier des mutants et des souches recombinantes.

14. Procédé selon l'une des revendications 1 à 13, dans lequel le procédé est conduit en continu, en semi continu ou par lots.

15. Procédé selon l'une des revendications précédentes, dans lequel l'étape de microfiltration, au moins au nombre de une, est exécutée selon la technique à écoulement transversal ou technique dite "Cross-flow".

16. Procédé selon l'une des revendications précédentes, dans lequel une culture phototrope de bactéries halophiles est utilisée comme culture de départ pour la fermentation.
